# EUROPÄISCHE PATENTANMELDUNG

(11) **EP 3 187 261 A1**
(43) Veröffentlichungstag der Anmeldung: **05.07.2017**
(21) Anmeldenummer: 15203119.1
(22) Anmeldetag: 30.12.2015
(51) Int. Cl.: B01J 23/30, B01J 37/02, C07C 319/08, C07C 321/04, B01J 35/02, B01J 37/00, B01J 21/04

(54) **VERFAHREN ZUR HERSTELLUNG EINES EIN ALKALIMETALL UND EINES ÜBERGANGSMETALL IN OXIDIERTER FORM ENTHALTENDEN KATALYSATORS**

(71) Anmelder: Evonik Degussa GmbH, 45128 Essen (DE)
(72) Erfinder: FONFE, Benjamin, 60318 Frankfurt (DE); DÜRR, Nadine, 64665 Alsbach (DE); JAKOB, Harald, 63594 Hasselroth (DE); PASHIGREVA, Anastasia, Town and Country, MO 63017 (US); GUTIERREZ, Oliver, 80805 München (DE); LERCHER, Johannes A., 85521 Ottobrunn (DE)

(57) **Zusammenfassung**

Die vorliegende Erfindung betrifft ein Verfahren zur Herstellung eines mindestens ein Alkalimetall und mindestens ein Übergangsmetall in oxidierter Form enthaltenden geträgerten Katalysators, umfassend die Schritte a) Bereitstellen einer Lösung mit mindestens einer ein Alkalimetall enthaltenden Verbindung und mindestens einer ein Übergangsmetall in oxidierter Form enthaltenden Verbindung, wobei das Alkalimetall und das Übergangsmetall Bestandteil derselben Verbindung sein können, b) Aufbringen der Lösung aus Schritt a) auf ein pulverförmiges Trägermaterial mit einem Partikeldurchmesser von weniger als 1000 µm, und c) Formen des aus Schritt b) erhaltenen beladenen Trägermaterials, ein nach dem erfindungsgemäßen Verfahren erhaltener oder erhältlicher Katalysator, und die Verwendung eines erfindungsgemäßen Katalysators und/oder eines nach dem erfindungsgemäßen Verfahren erhaltenen oder erhältlichen Katalysators in der Herstellung eines Alkylmercaptans.

## Beschreibung

Die vorliegende Erfindung betrifft ein Verfahren zur Herstellung eines Katalysators, der mindestens ein Alkalimetall und mindestens ein Übergangsmetall in oxidierter Form enthält, einen nach dem erfindungsgemäßen Verfahren erhaltenen oder erhältlichen Katalysator und desen Verwendung in der Herstellung eines Alkylmercaptans.

Alkylmercaptane, die im Rahmen der vorliegenden Erfindung auch gleichbedeutend mit dem Begriff Alkanthiol verwendet werden, sind organisch-chemische Verbindungen mit mindestens einer aliphatisch gebundenen Thiolgruppe (-SH) als funktioneller Gruppe. Formell betrachtet entsprechen Alkylmercaptane daher Alkoholen, deren Sauerstoffatom durch ein Schwefelatom ersetzt wurde. Neben ihrem vielfältigen direkten Anwendungspotential besteht die Bedeutung von Alkylmercaptanen in der organischen Chemie vor allem darin, dass sie wichtige Zwischenstufen in der Synthese anderer organischer Verbindungen mit entsprechender Wertschöpfung darstellen. Beispiele hierfür sind die Herstellung von Methionin sowie von Dimethyldisulfid und Dimethylsulfon durch Umsetzung von Methylmercaptan, welches das industriell bedeutendste Alkylmercaptan darstellt.

Industriell werden Alkylmercaptane typischerweise durch Reaktion von Alkylalkoholen mit Schwefelwasserstoff in Gegenwart eines auf Aluminiumoxid basierenden heterogenen Katalysators hergestellt. Diese Reaktion wird gewöhnlich als Gasphasenreaktion bei einer Temperatur zwischen 300 und 500°C sowie einem Druck zwischen 1 und 25 bar durchgeführt. Das aus dieser Reaktion erhaltene Produktgemisch enthält neben dem gewünschten Alkylmercaptan auch nicht umgesetzte Ausgangsverbindungen und Nebenprodukte, wie Dialkylsulfide und Dialkylether. Üblicherweise werden die nicht umgesetzten Ausgangsverbindungen, insbesondere der toxische Schwefelwasserstoff, unmittelbar nach ihrer Abtrennung aus dem Produktgemisch wieder in die Reaktion zurückgeführt, um die Gesamtausbeute bezogen auf die jeweilige Ausgangsverbindung zu erhöhen.

In der Regel wird das gewünschte Alkylmercaptan durch Auskondensieren aus dem Produktgemisch abgetrennt. Diese Trennung und insbesondere der mit der Kühlung des Produktgemisches verbundene Energieaufwand stellen einen großen Kostenfaktor dar. Um den Energieaufwand bei der Abtrennung des Alkylmercaptans vom Produktgemisch so gering wie möglich zu halten, wird daher eine möglichst hohe Ausbeute und Selektivität für die Bildung des Alkylmercaptans angestrebt.

Grundsätzlich können Ausbeute und Selektivität für die Bildung des betreffenden Alkylmercaptans sowohl durch eine Erhöhung des Molverhältnisses von Alkylalkohol zu Schwefelwasserstoff als auch durch die Wahl des eingesetzten Katalysators verbessert werden.

Zur Erzielung möglichst hoher Alkylmercaptan-Ausbeuten werden in der Praxis die Reaktanden Schwefelwasserstoff und Alkylmercaptan in einem zumindest äquimolaren Verhältnis, bevorzugt jedoch mit einem molaren Überschuss von Schwefelwasserstoff, umgesetzt. So werden beispielsweise in der Herstellung von Methylmercaptan Schwefelwasserstoff und Methanol in Molverhältnissen von 1:1 bis 10:1 miteinander umgesetzt. Ein hohes Molverhältnis von Schwefelwasserstoff zu Methanol bedeutet allerdings auch einen hohen Überschuss von Schwefelwasserstoff im Produktgemisch Die Rückführung großer Mengen nicht umgesetzten Schwefelwasserstoffs in den Prozess ist allerdings mit hohen Investitions- und Energiekosten verbunden. Zur Verminderung des hierfür erforderlichen Energieaufwandes sollte daher das Verhältnis von Schwefelwasserstoff zu Methanol nur wenig von 1 abweichen.

Der Möglichkeit die Ausbeute und Selektivität in der Herstellung von Alkylmercaptanen durch Erhöhen des Molverhältnisses von Schwefelwasserstoff und Alkylalkohol zu verbessern, sind also entsprechende Grenzen gesetzt. Die einzige verbleibende Möglichkeit zur Optimierung von Alkylmercaptan-Ausbeute und -Selektivität besteht daher in der Wahl geeigneter Katalysatoren. Hohe Ausbeuten und Selektivitäten für die Bildung von Alkylmercaptanen werden allgemein mit Katalysatoren erreicht, bei denen das katalytisch aktive Aluminiumoxid mit einem Alkaliwolframat als Promotor versetzt ist. Üblicherweise wird das Wolframat in Mengen von bis zu 25 Gewichtsprozent, bezogen auf das Gesamtgewicht des Katalysators, eingesetzt, um ein Mindestmaß an Aktivität für den eingesetzten Katalysators zu erreichen.

Durch Mehrfachimprägnierung eines Trägermaterials mit einer Lösung, die Cäsium und Wolfram in Verhältnissen von weniger als 2:1 enthält, wie in der WO 2005/021491 A1 bzw. der EP 0832878 A2 beschrieben, kann die Beladung des Katalysators mit Cäsiumwolframat auf bis 25 Gewichtsprozent und mehr, bezogen auf das Gesamtgewicht des Katalysators, gesteigert werden. Eine Erhöhung der Beladung des Katalysators mit Cäsiumwolframat auf über 25 Gewichtsprozent, bezogen auf das das Gesamtgewicht des Katalysators, führt zwar zu einer Steigerung der Selektivität für die Bildung des Alkylmercaptans, allerdings nimmt gleichzeitig auch die Aktivität des Katalysators ab. Wird die Beladungen des Katalysators mit Cäsiumwolframat auf 35 Gewichtsprozent und mehr, bezogen auf das Gesamtgewicht des Katalysators, erhöht, nehmen gleichzeitig die Selektivität und die Aktivität des Katalysators ab. Vergleichbare Effekte werden auch für die halogenidhaltigen Katalysatoren der WO 2006/015668 A1 und die halogenidfreien Katalysatoren der WO 2006/063669 A1, die jeweils durch Mehrfachimprägnierung hergestellt werden, beobachtet. Ein weiterer Nachteil in der Herstellung von Katalysatoren mittels Mehrfachimprägnierung besteht darin, dass Cäsium und Wolfram nicht gleichmäßig über den Querschnitt der üblicherweise in der Technik als Katalysatorträger eingesetzten Formkörper verteilt werden können. Eine gleichmäßige Verteilung der katalytisch aktiven Komponenten über den Querschnitt des Katalysatorträgers wird jedoch als erforderliches Kriterium für Katalysatoren mit hoher Aktivität und Selektivität der Herstellung von Alkylmercaptanen erachtet.

Gemäß der WO 2013/092129 A1 werden Katalysatoren mit einer gleichmäßigen Verteilung von Alkalimetall und Wolfram über den gesamten Formkörperquerschnitt dadurch erhalten, indem das Trägermaterial nicht imprägniert sondern mit einer oxidischen Wolframverbindung und mindestens einer separaten, also nicht in der Wolframverbindung enthaltenen, Alkalimetallverbindung vermischt und die derart erhaltene Katalysatormasse anschließend geformt wird. Die nach diesem Verfahren erhaltenen Katalysatoren haben eine Beladung mit Alkalimetallwolframat von über 45 Gewichtsprozent, bezogen auf das Gesamtgewicht des Katalysators. Dadurch können Umsatz und Selektivität für die katalysierte Bildung von Methylmercaptan weiter gesteigert werden. Im Vergleich dazu werden keine positiven Effekte auf Ausbeute und Selektivität für mittels Imprägnierung hergestellten Katalysatoren beobachtet, die eine Beladung mit Alkalimetallwolframat von 45 Gewichtsprozent und mehr, bezogen auf das Gesamtgewicht des Katalysators, aufweisen. Die mit den Katalysatoren der WO 2013/092129 A1 erzielten hohen Ausbeuten und Selektivitäten in der Herstellung von Methylmercaptan halten allerdings nicht lange an. Vielmehr wird eine rasche Abnahme des Methanol-Umsatzes und der Methylmercaptan-Selektivität beobachtet, die von einer Zunahme der Bildung des Nebenproduktes Dimethylether begleitet wird.

Es bestand daher Bedarf an einem Verfahren zur Bereitstellung von Katalysatoren, die über einen längeren Zeitraum eine hohe Selektivität für die Bildung von Alkylmercaptanen aus Alkylalkoholen und Schwefelwasserstoff erzielen.

Erfindungsgemäß wird diese Aufgabe dadurch gelöst, indem ein geträgerter Katalysator durch eine Kombination aus Aufbringen einer mindestens ein Alkalimetall und mindestens ein Übergangsmetall in oxidierter Form enthaltenden Lösung auf einen pulverförmigen Trägerkörpers mit einem Partikeldurchmesser von weniger als 1000 µm und anschließender Formgebung.

Gegenstand der vorliegenden Erfindung ist daher ein Verfahren zur Herstellung eines mindestens ein Alkalimetall und mindestens ein Übergangsmetall in oxidierter Form enthaltenden geträgerten Katalysators, umfassend die Schritte
a) Bereitstellen einer Lösung mit mindestens einer ein Alkalimetall enthaltenden Verbindung und mindestens einer ein Übergangsmetall in oxidierter Form enthaltenden Verbindung, wobei das Alkalimetall und das Übergangsmetall Bestandteil derselben Verbindung sein können,
b) Aufbringen der Lösung aus Schritt a) auf ein pulverförmiges Trägermaterial mit einem Partikeldurchmesser von weniger als 1000 µm, und
c) Formen des aus Schritt b) erhaltenen beladenen Trägermaterials.

Es wird vermutet, dass eine hohe Dispersion der für die Bildung von Alkylmercaptanen katalytisch aktiven Phase eine hohe Selektivität für die Bildung des Alkylmercaptans begünstigt. Die in Schritt b) des erfindungsgemäßen Verfahrens auf das Trägermaterial liegen entweder bereits als Oxide bzw. in oxidierter Form vor oder werden durch eine anschließende Behandlung, bevorzugt durch Calcination in das entsprechende Oxid oder in die entsprechende oxidische Form umgewandelt. Eine hohe Dispergierung der katalytisch aktiven Phase wird in optimaler Weise durch eine möglichst hohe Dispergierung des entsprechenden Vorläufers für das Oxid der katalytisch aktiven Phase erzielt. Es wird vermutet, dass es sich bei dieser Dispergierung um eine Vermischung von Alkalimetall und Übergangsmetall auf ionischer Ebene handelt. Weiter wird vermutet, dass eine verbesserte Verteilung des aufgebrachten Alkalimetalls und Übergangsmetalls durch die anschließende Formung des in Schritt b) beladenen Trägermaterials erzielt wird. Um eine möglichst gute Verteilung durch diese mechanische Behandlung zu bewirken, wird ein pulverförmiges Trägermaterial mit möglichst kleinem Partikeldurchmesser eingesetzt. Vorzugsweise hat das pulverförmige Trägermaterial einen Partikeldurchmesser von weniger als 500 µm, besonders bevorzugt von weniger als 250 µm oder sogar weniger als 100 µm.

In einer Ausführungsform des erfindungsgemäßen weist das pulverförmige Trägermaterial einen Partikeldurchmesser von weniger als 250 µm auf.

Besonders gute Ergebnisse werden mit einem pulverförmigen Trägermaterial aus Aluminiumoxid, bevorzugt gamma-Aluminiumoxid, erhalten. Denn Aluminiumoxid und insbesondere gamma-Aluminiumoxid weist bereits selber katalytische Aktivität für die Umsetzung von Alkylalkoholen mit Schwefelwasserstoff zu Alkylmercaptanen auf.

In einer Ausführungsform des erfindungsgemäßen Verfahrens ist daher das pulverförmige Trägermaterial Aluminiumoxid.

Hinsichtlich der in Schritt a) bereitgestellten Lösung unterliegt das erfindungsgemäße Verfahren keinen Beschränkungen. Daher können im erfindungsgemäßen Verfahren sowohl eine einzelne Lösung mit einer einzigen Verbindung, die sowohl das Alkalimetall und das Übergangsmetall enthält, als auch eine Lösung mit mehreren Verbindungen, die ein Alkalimetall oder mehrere Alkalimetalle bzw. ein Übergangsmetall in oxidierter Form oder mehrere Übergangsmetalle in oxidierter Form eingesetzt werden. Wenn mehrere Lösungen eingesetzt werden, dann können diese entweder dasselbe Alkalimetall bzw. Übergangsmetall in oxidierter Form oder dieselben Alkalimetalle bzw. Übergangsmetalle in oxidierter Form oder unterschiedliche Alkalimetalle bzw. Übergangsmetalle in oxidierter Form enthalten. Die Bereitstellung einer Lösung in Schritt a) erfolgt durch Auflösen von Alkalimetallverbindungen wie Hydroxiden, Carbonaten, Oxalaten, Acetaten oder ähnlichen von beispielsweise Natrium, Kalium oder Cäsium, und Verbindungen mit einem Übergangsmetall in oxidierter Form wie Wolframsäure, Wolframtrioxid, Alkaliwolframat oder Ammoniumwolframat in protischen Medien, wie Wasser, Ethanol oder auch Ammoniak oder wässrigen Lösungen von Ammoniak. Sind das Alkalimetall und das in oxidierter Form vorliegende Übergangsmetall Bestandteil derselben Verbindung, handelt es sich bei dieser Verbindung bevorzugt um ein Alkaliwolframat, insbesondere um Natrium-, Kalium- oder Cäsiumwolframat.

Auch hinsichtlich des Aufbringens der in Schritt a) bereitgestellten Lösung unterliegt das erfindungsgemäße Verfahren keinen Beschränkungen. Vorteilhafterweise erfolgt das Aufbringen der in Schritt a) bereitgestellten Lösung durch Imprägnieren des pulverförmigen Trägermaterials mit der Lösung. Denn dies stellt eine besonders einfach durchführbare Möglichkeit für das Aufbringen der Lösung auf das pulverförmige Trägermaterial dar. Das Aufbringen der Lösung auf das pulverförmige Trägermaterial wird im Rahmen der vorliegenden Erfindung auch als Beladen des Trägermaterial mit der Lösung, dem Alkalimetall, dem Übergangsmetall, den katalytisch aktiven Elementen oder artverwandt bezeichnet.

In einer Ausführungsform des erfindungsgemäßen Verfahrens wird daher in Schritt b) die Lösung durch Imprägnieren auf das pulverförmige Trägermaterial aufgebracht.

Durch eine mehrstufige Imprägnierung mit einer oder mehreren identischen oder unterschiedlichen Imprägnierlösungen können entweder unterschiedliche Alkalimetalle und/oder Übergangsmetalle oder eine höhere Beladung desselben Alkalimetalls und/oder desselben Übergangsmetalls auf das pulverförmige Trägermaterial aufgebracht werden. Die Imprägnierung kann daher beispielsweise zweistufig oder dreistufig erfolgen.

In einer bevorzugten Ausführungsform des erfindungsgemäßen Verfahrens wird daher in Schritt b) die Lösung durch mehrstufiges Imprägnieren auf das pulverförmige Trägermaterial aufgebracht.

Für die Herstellung von Alkylmercaptanen aus Alkylalkoholen und Schwefelwasserstoff sind geträgerte Katalysatoren, insbesondere mit Aluminiumoxid und insbesondere gamma-Aluminiumoxid als Trägermaterial, auf Basis von Wolfram in oxidierter Form besonders gut geeignet. Besonders gut für die Herstellung von Alkylmercaptanen aus Alkylalkoholen und Schwefelwasserstoff sind solche geträgerten Katalysatoren der vorstehend beschriebenen Art geeignet, bei denen das Alkalimetall Natrium, Kalium oder Cäsium ist. Besonders bevorzugt handelt es sich im erfindungsgemäßen Verfahren bei dem Alkalimetall um Cäsium.

In einer Ausführungsform des erfindungsgemäßen Verfahrens ist daher das Übergangsmetall Wolfram.

In einer weiteren Ausführungsform des erfindungsgemäßen Verfahrens ist das Alkalimetall Natrium, Kalium oder Cäsium.

Im Gegensatz zu den aus dem Stand der Technik bekannten Verfahren, beispielsweise dem Verfahren der WO 2006/063669 A1, werden im erfindungsgemäßen Verfahren bevorzugt nur solche Alkalimetall- und Übergangsmetall enthaltende Verbindungen eingesetzt, die keine Halogene enthalten. Denn es hat sich gezeigt, dass Halogen enthaltende Katalysatoren in der Herstellung von Alkylmercaptanen zu einer teilweise starken Korrosion der Reaktorrohre führen.

In einer Ausführungsform des erfindungsgemäßen Verfahrens sind daher die mindestens eine ein Alkalimetall enthaltende Verbindung und die mindestens eine ein Übergangsmetall in oxidierter Form enthaltende Verbindung halogenfrei.

Die erfindungsgemäß durchgeführte Formgebung bewirkt eine verbesserte Verteilung des auf das Trägermaterial aufgebrachten Alkalimetalls und des Übergangsmetalls. Prinzipiell eignet sich jede denkbare Formgebung des in den vorangegangenen Schritten beladenen pulverförmigen Trägermaterials zu diesem Zweck, solange gewährleistet wird, dass durch die jeweilige Formgebung eine verbesserte Verteilung des auf das Trägermaterial aufgebrachten Alkalimetalls und des Übergangsmetalls erzielt wird. Einfach durchführbare Möglichkeiten, die diese Vorgabe erfüllen, sind Extrusion und Tablettierung, wobei diese Schritte gemäß den üblichen Vorgehensweisen für eine Extrusion oder Tablettierung von Katalysatorpartikeln durchgeführt werden können.

In einer Ausführungsform des erfindungsgemäßen Verfahrens erfolgt die Formung in Schritt c) durch Extrusion oder Tablettierung.

Eine besonders gute Verteilung des auf das Trägermaterial aufgebrachten Alkalimetalls und des Übergangsmetalls wird dann erzielt, wenn vor der Formung das beladene Trägermaterial den Schritten der Trocknung und/oder Calcinierung sowie der Durchmischung des beladenen Trägermaterials unterworfen wird. Erfolgt das Aufbringen in Schritt b) des erfindungsgemäßen Verfahrens durch Imprägnierung, wird das beladene Trägermaterial zunächst von der Imprägnierlösung getrennt, was bevorzugt durch Filtration erfolgt. Da das von der Imprägnierlösung getrennte beladene Trägermaterial noch Restfeuchtigkeit enthält, wird es dann bevorzugt einer Trocknung und/oder Calcinierung unterworfen. Die Trocknung erfolgt bei einer Temperatur, die oberhalb der Siedetemperatur des zur Bereitstellung der Imprägnierlösung bzw. der Imprägnierlösung verwendeten Mediums, bevorzugt Wasser, liegt. In den meisten Fällen erfolgt die Trocknung daher bei Temperaturen von mehr als 100°C. Bevorzugt erfolgt die Trocknung durch Erhöhung der Temperatur auf ca. 120°C mit einer Aufheizrate von ca. 1°C/min. Diese Temperatur wird dann für mehrere Stunden, bevorzugt für bis zu 3 oder mehr Stunden, gehalten. Gegebenenfalls kann der Trocknung auch eine Vortrocknung bei einer Temperatur von weniger als 100°C, bevorzugt bei ca. 60°C, vorangehen, um leichtflüchtige Komponenten des zur Herstellung der Imprägnierlösung verwendeten Mediums, bevorzugt Ammoniak, zu entfernen. Zur Überführung des Alkalimetalls und des Übergangsmetalls in eine oxidische Form bzw. zur Sicherstellung, dass das Alkalimetall und das Übergangsmetall in die oxidische Form überführt worden sind, schließt sich der Trocknung eine Calcinierung an. Bevorzugt wird die Calcinierung bei Temperaturen von 400°C und mehr durchgeführt. Bevorzugt wird die Calcinierung so durchgeführt, dass die Temperatur von dem Wert bei der Trocknung mit einer Aufheizrate von ca. 5°C/min auf die endgültige Calcinierungstemperatur erhöht wird. Diese Temperatur wird dann für mehrere Stunden, bevorzugt für bis zu 3 oder mehr Stunden, gehalten.

Bevorzugt wird das beladene Trägermaterial, insbesondere dann wenn es zusammengebacken ist, beispielsweise nach der Abtrennung der Imprägnationsflüssigkeit durch Filtration, durchmischt. Liegt beispielsweise nach einer Filtration ein Filterkuchen vor, dann wird dieser Filterkuchen nach dem Calcinieren gebrochen und gemahlen. Im Rahmen des erfindungsgemäßen Verfahrens dient das Mahlen primär zur Durchmischung des beladenen Trägermaterials, um eine möglichst gleichmäßige Verteilung der auf das Trägermaterial aufgebrachten Elemente zu erzielen. Daher ist es im Rahmen des erfindungsgemäßen Verfahrens nicht erforderlich, dass mit dem Mahlvorgang auch eine weitere Zerkleinerung der beladenen Partikel erzielt wird.

Alternativ oder ergänzend kann der Filterkuchen auch vor dem Trocknen und/oder Calcinieren durchmischt werden, bevorzugt mit Hilfe eines Kneters, beispielsweise eines Laborchargenkneters. Hinsichtlich der Reihenfolge der Schritte i) Trocknung und/oder Calcinierung und ii) Durchmischung sowie der Anzahl der Durchführung dieser Schritte unterliegt das erfindungsgemäße Verfahren daher keiner Beschränkung.

In einer Ausführungsform des erfindungsgemäßen Verfahrens umfasst die Formung in Schritt zusätzlich die der Formung vorangehenden Schritte
i) Trocknung und/oder Calcinierung des aus Schritt b) erhaltenen beladenen Trägermaterials, und
ii) Durchmischung des beladenen Trägermaterials.

In einer bevorzugten Ausführungsform des erfindungsgemäßen Verfahrens erfolgt die Durchmischung durch Mahlen.

Die Schritte i) und ii) führen in Kombination mit den Schritten a) bis c) des erfindungsgemäßen Verfahrens zur Ausbildung einer katalytischen Aktivmasse auf dem Trägermaterial. Zur Erhöhung der Beladung des Trägermaterials mit der katalytischen Aktivmasse wird das aus Schritt c) erhaltene beladene Trägermaterial daher bevorzugt erneut den Schritten a) bis c) des erfindungsgemäßen Verfahrens unterworfen.

In einer weiteren Ausführungsform des erfindungsgemäßen Verfahrens werden daher die Schritte a) bis c) mit dem aus Schritt c) erhaltenen beladenen Trägermaterial wiederholt.

Der eigentlich in der Herstellung von Alkylmercaptanen aus Alkylalkoholen und Schwefelwasserstoff vorliegende Katalysator ist sulfidischer Natur. Bevorzugt wird daher der nach dem erfindungsgemäßen Verfahren erhaltene oder erhältliche Katalysator nach der Formung einem Sulfidierungsschritt unterworfen. Im einfachsten Fall wird der Sulfidierungsschritt mit dem in einen Reaktor eingefüllten Katalysator vorgenommen. Bevorzugt wird die Sulfidierung durch Behandlung des nach dem erfindungsgemäßen Verfahren erhaltenen oder erhältlichen Katalysators mit Schwefelwasserstoff durchgeführt.

Die nach dem erfindungsgemäßen Verfahren hergestellten oder erhältlichen Katalysatoren sowie die erfindungsgemäßen Katalysatoren eignen sich für die Herstellung von Alkylmercaptanen aus Alkylalkoholen und Schwefelwasserstoff.

Ein weiterer Gegenstand der vorliegenden Erfindung ist ein nach dem erfindungsgemäßen Verfahren erhaltener oder erhältlicher Katalysator.

Ferner ist ein weiterer Gegenstand der vorliegenden Erfindung auch die Verwendung eines nach dem erfindungsgemäßen Verfahren erhaltenen oder erhältlichen Katalysators und/oder eines erfindungsgemäßen Katalysators in der Herstellung eines Alkylmercaptans durch Umsetzung eines Alkylalkohols mit Schwefelwasserstoff. Im Rahmen der vorliegenden Erfindung wird der Ausdruck Alkylalkohol gleichbedeutend mit Alkanol verwendet und bezeichnet einen Alkohol bei dem die Hydroxygruppe direkt an ein aliphatisches Kohlenstoffatom gebunden ist.

Hinsichtlich des umzusetzenden Alkylalkohols unterliegt die erfindungsgemäßen Verwendung keinen Beschränkungen, solange gewährleistet ist, dass die eingesetzten Alkylalkohole bei den in der Herstellung von Alkylmercaptanen typischerweise angewandten Reaktionsbedingungen keinen Nebenreaktionen unterliegen. In der Regel ist dies durch Verwendung von Alkylalkoholen mit 1 bis 4 Kohlenstoffatomen, insbesondere linearen Alkylalkoholen mit 1 bis 4 Kohlenstoffatomen, sichergestellt.

In einer Ausführungsform der erfindungsgemäßen Verwendung hat der Alkylalkohol daher 1 bis 4 Kohlenstoffatome.

Bevorzugt handelt es sich bei dem eingesetzten Alkylalkohol um Methanol.

### Beispiele:

### Beispiel 1 (Vergleichsbeispiel):

Wie in der Beispiel 2 (Vergleichsbeispiel) auf Seite 17 der WO 2013/092129 A1 beschrieben wurden 200 g kugelförmiges Aluminiumoxid mit einem Partikeldurchmesser von 2 bis 5 mm (Spheralite 501 A der Firma Axens mit einer spezifischen Oberfläche von 303 m²/g, einem Porenvolumen von 45 ml/100 g und einer Schüttdichte von 815 kg/m³) in einer dreistufigen Imprägnierung mit 17,8 Gew.-% WO₃ und 17,3 Gew.-% Cs₂O bezogen auf die Gesamtmasse des Katalysators beladen. Hierzu wurde wie folgt vorgegangen: 62,2 g Wolframsäure wurden in 130,4 g Ammoniaklösung suspendiert und durch Rühren für etwa 30 Minuten aufgelöst. 89,3 g einer Lösung von Cäsiumhydroxid in Wasser (70 Gew.-%) wurden zu dieser Lösung hinzugegeben, und die resultierende Lösung wurde für etwa 23 bis 24 Stunden gerührt. Das Aluminiumoxid wurde in einem Glasgefäß vorgelegt, das auf 150 mbar evakuiert wurde. Durch Öffnen des Hahns wurde die Imprägnierlösung solange abgesaugt, bis über dem gesamten Aluminiumoxid ein Überstand an Imprägnierlösung von etwa 4,5 cm bestand Nach Belüften des Glasgefäßes wurde der Träger in der Lösung für etwa 15 Minuten inkubiert. Anschließend wurde die Lösung abgelassen, und der erhaltene Katalysator wurde durch Hindurchleiten eines Luftstroms von 200 NI/h (Volumenstrom im Normzustand bei 0°C und 1,013 bar absolut nach DIN 1343) für 1 Stunde vorgetrocknet, wobei noch anhaftende Imprägnierlösung in den Vorlagenbehälter gespült wurde. Anschließend wurde der Katalysator unter einem Luftstrom von 60 m³/h mit einer Aufheizrate von 1°C/min auf 120°C erwärmt und 3 Stunden bei dieser Temperatur getrocknet. Danach wurde die Temperatur mit einer Aufheizrate von 5°C/min auf 455°C erhöht, und der Katalysator 3 Stunden bei dieser Temperatur calciniert. Zur Durchführung der zweiten Imprägnierung wurde eine bereits für den ersten Schritt beschriebene Imprägnierlösung angesetzt und in gleicher Weise durch Vakuumimprägnation auf den bereits beladenen Katalysator, welcher aus der ersten Imprägnierung erhalten worden war, aufgebracht. Die Vortrocknung des aus der zweiten Imprägnierung erhaltenen Katalysators bei Raumtemperatur, gefolgt von der dreistündigen Trocknung bei 120°C und der anschließenden Calcinierung bei 455°C für 3 Stunden erfolgte wie bereits beschrieben. Zur Durchführung der dritten Imprägnierung wurde ebenso verfahren.

### Beispiel 2 (Vergleichsbeispiel):

Wie in Beispiel 6 auf den Seiten 18 und 19 der WO 2013/092129 A1 beschrieben wurden 1,05 kg eines pulverförmigen Aluminiumoxids mit einem Partikeldurchmesser von 7 bis 15 µm (Spheralite 509 A der Firma Axens mit einer spezifischen Oberfläche von 335 m²/g, einem Porenvolumen von 56 ml/100 g und einer Schüttdichte von 840 kg/m³) nacheinander mit fester Wolframsäure und einer Calciumhydrodixlösung vermischt. Hierzu wurde wie folgt vorgegangen: 1,05 kg Spheralite 509 A und 537,9 g Wolframsäure wurden in einem Laborchargenkneter (Coperion LUK 2.5, Weinheim, Stuttgart, Deutschland) bei 40 Umdrehungen/min der Knethaken und 11 Umdrehungen/min der Austragsschnecke (rückwärtsgerichtet) gemischt, wobei der in dem Kneter befindliche Trog mit einem Kryostat auf 10 °C gekühlt wurde. Anschließend wurden 740,5 g einer 70 Gew.-%igen wässrigen Cäsiumhydroxidlösung innerhalb 1 min unter ständigem Durchmischen hinzugegeben, wobei die Temperatur kurzzeitig um 30 bis 40 °C stieg. 10 Minuten nach beendeter Zugabe wurden 127,5 g vollentsalztes Wasser und anschließend 175 g einer kolloidalen Kieselsäuredispersion (Lithosol 1530, Zschimmer & Schwarz GmbH & Co. KG, Lahnstein, Deutschland) hinzugegeben. Das erhaltene Gemisch wurde für weitere 10 Minuten durchmischt, bevor eine Mischung von 30 g eines hochpolymeren Polysaccharids (Zusoplast PS 1, Zschimmer & Schwarz GmbH & Co. KG, Lahnstein, Deutschland) und 30 g Hydroxyethylcellulose (Tylose H 10000 P2 ShinEtsu, Tokio, Japan) hinzugegeben wurden. Die Binder wurden unter ständigem Kneten der Masse für 120 Minuten quellen gelassen. Anschließend wurden 15 g einer nichtionischen Wachsdispersion (Zusoplast WE8, Zschimmer & Schwarz GmbH & Co. KG, Lahnstein, Deutschland) hinzugefügt. Nach einer Gesamtknetzeit von 190 Minuten wurde mit der Extrusion begonnen, wozu bei gleichbleibender Kneterdrehzahl die Drehrichtung der Schnecke auf Fördern umgestellt wurde und die Drehzahl der Schnecke auf 13 Umdrehungen/min erhöht wurde. Als Presswerkzeug wurde ein Aufsatz mit vier horizontalen Bohrungen mit einem jeweiligen Durchmesser von 3,2 mm verwendet. Es wurden zwei horizontal schneidende Schneiddrähte mit 400 Umdrehungen/min betrieben, um Extrudate einer Länge von etwa 3,2 mm zu erhalten. Der Drüsendruck betrug 12,7 bar. Die geschnittenen Extrudate wurden auf ein Trockenband fallen gelassen und bei 60 °C vorgetrocknet, bevor sie in einem Muffelofen mit einer Aufheizrate von 1 °C/min auf 120 °C erwärmt wurden und 3 Stunden bei dieser Temperatur getrocknet wurden. Direkt im Anschluss wurden die getrockneten Extrudate calciniert, indem die Temperatur mit einer Aufheizrate von 5 °C/min auf 455 °C erhöht wurden und diese Temperatur 3 Stunden lang gehalten wurde.

### Beispiel 3 (erfindungsgemäß):

150 g eines pulverförmigen Aluminiumoxids mit einem Partikeldurchmesser von 7 bis 15 µm (Spheralite 509 A der Firma Axens mit einer spezifischen Oberfläche von 335 m²/g, einem Porenvolumen von 56 ml/100 g und einer Schüttdichte von 840 kg/m³) wurden mit 17,8 Gew.-% WO₃ und 17,3 Gew.-% Cs₂O bezogen auf die Gesamtmasse des Katalysators beladen. Hierzu wurde wie folgt vorgegangen: 63,8 g Wolframsäure wurden in 127,7 g einer 25%-gen Ammoniaklösung aufgelöst. Zu dieser Lösung wurden 121,3 g einer Lösung von Cäsiumhydroxid in Wasser (50,48 Gew.-%) hinzugegeben, und dann wurde die Lösung für 24 Stunden gerührt. Anschließend wurde die Lösung durch Zugabe von Wasser auf ein Volumen von 243 ml aufgefüllt. Die so erhaltene Lösung wurde als Imprägnierlösung bezeichnet. Die Imprägnierlösung wurde in einem Glasgefäß vorgelegt und unter ständigem Rühren wurde das Aluminiumoxid hinzugegeben. 15 Minuten nach beendeter Zugabe wurde die Suspension über einem Blaubandfilter in einer Nutsche abfiltriert. Zur Entfernung der Restfeuchte wurde der Filterkuchen bei 120°C für 3 Stunden getrocknet. Dann wurde die Temperatur mit einer Aufheizrate von 5°C/min auf 455°C erhöht und der Filterkuchen für 3 Stunden bei dieser Temperatur calciniert. Nach dem Calcinieren wurde der Filterkuchen gebrochen und gemahlen. Das so erhaltene Pulver wurde erneut wie vorstehend beschrieben unter Verwendung einer auf gleiche Weise hergestellten Imprägnierlösung imprägniert. Trocknung und Calcination erfolgten ebenfalls wie vorstehend beschrieben. Nach dem Calcinieren wurde der Filterkuchen gebrochen und gemahlen. Das so erzeugte Katalysatorpulver wurde anschließend in einer Tablettenpresse zu Tabletten verpresst.

### Beispiel 4 (Testung der Katalysatoren):

Die in den Beispielen 1 bis 3 hergestellten Katalysatoren wurden hinsichtlich seiner Leistungscharakteristika in der Synthese von Methylmercaptan aus Schwefelwasserstoff und Methanol untersucht. Die Umsetzung von Schwefelwasserstoff mit Methanol zu Methylmercaptan wurde in einem Edelstahlrohr von 18 mm Durchmesser und einer Länge von 500 mm durchgeführt. Es wurde jeweils eine Katalysatorschüttung mit einem Volumen von 76 ml eingesetzt, welche jeweils beiderseits durch Interschüttungen aus Glaskugeln im Reaktionsrohr fixiert wurde. Das Reaktionsrohr wurde über einen Doppelmantel mit einem Thermoöl auf die unterschiedlichen, in der Tabelle 1 angegebenen Reaktionstemperaturen im Bereich von 300 bis 360°C erwärmt, die für einen Methanolumsatz von ungefähr 90% erforderlich sind. Die weiteren Versuchsbedingungen waren: GHSV (gas hourly space velocity) = 1300 h⁻¹ (bezogen auf Normbedingungen bei 0°C und 1,013 bar gemäß DIN 1343), LHSV (liquid hourly space velocity) = 0,4 h⁻¹, das Massenverhältnis von Schwefelwasserstoff zu Methanol betrug 1,9, und der Druck lag bei 9 bar. Die jeweils erhaltenen Reaktionsgemische, enthaltend die Produkte Methylmercaptan, Dimethylsulfid, Dimethyldisulfid und Dimethylether sowie die nicht umgesetzten Edukte Methanol und Schwefelwasserstoff, wurden online gaschromatographisch untersucht. Die Versuchsparameter für die Umsetzungen mit den jeweiligen Katalysatoren und die diesbezüglichen Messergebnisse sind in der untenstehenden Tabelle zusammengefasst.

**Tabelle 1: Übersicht über die Versuchsergebnisse.**

| **Katalysator** | **Zeit [h]** | **Temperatur [°C]** | **Umsatz [%]** | **Selektivität [%]** | **Beladung [Gew.-%]** | |
|---|---|---|---|---|---|---|
| | | | | | **WO₃** | **Cs₂O** |
| **Beispiel 1 (Vergleichs-Beispiel)** | 65 | 357 | 89,9 | 96 | | |
| | 130 | 352 | 90,2 | 95,8 | 17,3 | 17,8 |
| | 200 | 352 | 90,4 | 95,8 | | |
| | 230 | 352 | 90,4 | 96 | | |
| | 65 | 325 | 90,1 | 96,5 | | |
| **Beispiel 2 (VergleichsBeispiel)** | 130 | 332 | 90 | 96,4 | | |
| | 200 | 336 | 89,9 | 92,2 | 25,8 | 22,5 |
| | 230 | 336 | 89,9 | 95,9 | | |
| | 235 | 336 | 89,9 | 95,7 | | |
| | 280 | 336 | 89,9 | 95,6 | | |
| | 65 | 320 | 89,9 | 96,2 | | |
| | 130 | 325 | 88,6 | 96,2 | | |
| **Beispiel 3** | 200 | 328 | 90 | 96,1 | 17,3 | 17,8 |
| | 230 | 329 | 90 | 96,1 | | |
| | 235 | 328 | 90 | 96,1 | | |
| | 280 | 328 | 90 | 96,1 | | |

Die Messergebnisse zeigen, dass der erfindungsgemäße Katalysator aktiver ist als der Katalysator des Beispiels 1 (Vergleichsbeispiel) und daher zur Erzielung eines Methanolumsatzes von 90% eine deutlich niedrigere Reaktionstemperatur als der Katalysator des Beispiels 1 (Vergleichsbeispiel) benötigt. Darüber hinaus erzielt der erfindungsgemäße Katalysator zusätzlich auch noch eine höhere Selektivität für die Bildung von Methylmercaptan als der Katalysator des Beispiels 1 (Vergleichsbeispiel).

Die Messergebnisse zeigen ferner, dass der erfindungsgemäße Katalysator auch aktiver ist als der Katalysator des Beispiels 2 (Vergleichsbeispiel). Dies ist umso erstaunlicher, wenn man berücksichtigt, dass der Katalysator des Beispiels 2 eine mehr als 37% höhere Beladung mit WO₃ und Cs₂O aufweist als der erfindungsgemäße Katalysator. Außerdem zeigen die Messergebnisse, dass der erfindungsgemäße Katalysator im Gegensatz zum Katalysator des Beispiels 2 langzeitstabil ist. Denn der erfindungsgemäße Katalysator hält die anfänglich erzielte Selektivität für die Bildung von Methylmercaptan nicht nur fast unvermindert aufrecht; vielmehr erzielt er nach längerer Betriebszeit sogar eine bessere Methylmercaptan-Selektivität als der Katalysator des Beispiels 2 (Vergleichsbeispiel).

## Patentansprüche

1. Verfahren zur Herstellung eines mindestens ein Alkalimetall und mindestens ein Übergangsmetall in oxidierter Form enthaltenden geträgerten Katalysators, umfassend die Schritte
a) Bereitstellen einer Lösung mit mindestens einer ein Alkalimetall enthaltenden Verbindung und mindestens einer ein Übergangsmetall in oxidierter Form enthaltenden Verbindung, wobei das Alkalimetall und das Übergangsmetall Bestandteil derselben Verbindung sein können,
b) Aufbringen der Lösung aus Schritt a) auf ein pulverförmiges Trägermaterial mit einem Partikeldurchmesser von weniger als 1000 µm, und
c) Formen des aus Schritt b) erhaltenen beladenen Trägermaterials.

2. Verfahren gemäß Anspruch 1, wobei das pulverförmiges Trägermaterial einen Partikeldurchmesser von weniger als 250 µm aufweist.

3. Verfahren gemäß Anspruch 1 oder 2, wobei das pulverförmige Trägermaterial Aluminiumoxid ist.

4. Verfahren gemäß einem beliebigen der Ansprüche 1 bis 3, wobei die Lösung in Schritt b) durch Imprägnieren auf das pulverförmige Trägermaterial aufgebracht wird.

5. Verfahren gemäß Anspruch 4, wobei die Lösung in Schritt b) durch mehrstufiges Imprägnieren auf das pulverförmige Trägermaterial aufgebracht wird.

6. Verfahren gemäß einem beliebigen der Ansprüche 1 bis 5, wobei das Übergangsmetall Wolfram ist.

7. Verfahren gemäß einem beliebigen der Ansprüche 1 bis 6, wobei das Alkalimetall Natrium, Kalium oder Cäsium ist.

8. Verfahren gemäß einem beliebigen der Ansprüche 1 bis 7, wobei die mindestens eine ein Alkalimetall enthaltende Verbindung und die mindestens eine ein Übergangsmetall in oxidierter Form enthaltende Verbindung halogenfrei sind.

9. Verfahren gemäß einem beliebigen der Ansprüche 1 bis 8, wobei die Formung in Schritt c) durch Extrusion oder Tablettierung erfolgt.

10. Verfahren gemäß einem beliebigen der Ansprüche 1 bis 9, wobei die Formung zusätzlich umfassend die der Formung vorangehenden Schritte
i) Trocknung und/oder Calcinierung des aus Schritt b) erhaltenen beladenen Trägermaterials, und
ii) Durchmischung des beladenen Trägermaterials.

11. Verfahren gemäß Anspruch 10, wobei die Durchmischung durch Mahlen erfolgt.

12. Verfahren gemäß einem beliebigen der Ansprüche 1 bis 11, wobei die Schritte a) bis c) mit dem aus Schritt c) erhaltenen beladenen Trägermaterial wiederholt werden.

13. Katalysator erhalten oder erhältlich nach einem Verfahren gemäß einem beliebigen der vorangehenden Ansprüche 1 bis 12.

14. Verwendung eines nach einem Verfahren gemäß einem beliebigen der Ansprüche 1 bis 12 erhaltenen oder erhältlichen Katalysators und/oder eines Katalysators gemäß Anspruch 13 in der Herstellung eines Alkylmercaptans durch Umsetzung eines Alkylalkohols mit Schwefelwasserstoff.

15. Verwendung gemäß Anspruch 14, wobei der Alkylalkohol 1 bis 4 Kohlenstoffatome hat.
